# EUROPEAN PATENT APPLICATION

(11) **EP 0 720 020 A1**
(43) Date of publication of application: **03.07.1996**
(21) Application number: 95309523.9
(22) Date of filing: 29.12.1995
(51) Int. Cl.: G01N 33/53, B01L 3/00, G01N 27/00, G01N 33/569

(54) **Apparatus and method for testing dried blood spots by the Western blot procedure**

(30) Priority: 30.12.1994 US 367046
(71) Applicant: ORTHO PHARMACEUTICAL CORPORATION, Raritan, NJ 08869 (US)
(72) Inventor: Pagels, William, Oceanport, NJ 07757 (US); Galvin, Phillip, Wilmington, Delaware 19803 (US); Rolon, Noel, Belle Mead, NJ 08502 (US); Egleston, Kim, Long Valley, NJ 07853 (US)
(74) Representative: Fisher, Adrian John

(57) **Abstract**

An apparatus and method for performing Western Blot testing of dried blood spots (DBSs) containing dried blood sample is disclosed. The apparatus comprises a Western Blot tray and means for agitating buffer, DBS(s) and a Western Blot strip. The troughs are shaped such that smaller amounts of blood sample and buffer may be used to achieve the same or higher concentration levels of blood sample to volume of buffer than ordinarily achieved by using standard Western Blot trays. A method for performina the Western Blot test in acccrdance with this invention is also disclosed.

## Description

### Field Of The Invention

The present invention relates generally to techniques for detecting a foreign element in a blood sample or similar and, more particularly, to a system for performing the Cambridge Biotech Western Blot test.

### Background Of The Invention

Acquired immunodeficiency syndrome (AIDS) is caused by a virus transmitted by sexual contact, exposure to blood or certain blood products, or transmitted from an infected mother to her fetus or child during the perinatal period. Human Deficiency Virus Type 1 (HIV-1) has been isolated from patients with AIDS or AIDS-related complex and from healthy persons at high risk form AIDS.

An Enzyme-Linked Immunosorbent Blot Technique which uses separate HIV proteins immobilized on nitrocellulose paper is the Western Blot assay. One example of such an assay is the Cambridge Biotech HIV-1 Western Blot Kit. This assay has been used to analyze serum or plasma samples from an individual found to be repeatably reactive. In this test, individual nitrocellulose strips are incubated with serum, plasma specimens or controls. If HIV-1 antibodies are present in the specimen during incubation, the antibodies will bind to the viral antigens. Visualization of the human immunoglobulins specifically bound to HIV-1 proteins is accomplished in situ using a series of reactions with goat anti-human IgG conjugated with biotin, avidin conjugated with horseradish peroxidase and the HRP substrate 4-chloro-1-naphthol. If antibodies to any of the major HIV-1 antigens are present in the specimen in sufficient concentration, bands corresponding to the position of one or more of the HIV-1 proteins will be seen.

With the advent of the in-home systems for the collection of blood and similar samples for the purpose of laboratory testing for the HIV-1 virus, it has become necessary to develop a simplified system for collecting such samples. One such system includes collecting blood samples on a test card such as the card disclosed in copending U.S. Patent Application Serial No. (unknown) entitled "Device and Test Kit For The Detection of AIDS", which application is incorporated herein by reference. The test cards typically include a filter paper section having areas designated and, preferably, outlined with dotted lines, on to which the user deposits blood samples. Each blood sample dries in the filter paper forming one or more areas of dried test blood. The filter paper containing the area of dried blood is typically punched out of the card for testing of the blood. These punched areas generally referred to as dried blood spots (DBS) are, under current testing techniques, preferably in the shape of approximately ¼" diameter circles. The ¼" size has been utilized primarily because two such DBSs provide a sufficient amount of blood sample with which to perform Western Blot (WB) testing. Inasmuch as the number of DBSs available from each test subject is limited (typically to three ¼" diameter DBSs per test card), the need exists for an apparatus and method to perform WB testing which requires less of the available blood sample.

Current techniques used to perform the WB assay with plasma or serum samples, employ standard commercially available Western Blot trays. Such trays generally include a plurality of parallel rectangularly shaped wells or troughs. This procedure also requires at least two DBS approximately ¼" in diameter, a nitrocellulose WB strip and 2 ml of buffer, all of which are deposited together in a single trough. The WB strip, DBS and buffer are agitated during incubation. The agitation is usually provided by tilting, rocking or rotating the tray.

Western Blot trays may vary in size and dimension. A standard WB tray typically comprises a multiplicity of parallel rectangular troughs in which WB strips are placed together with buffer and DBS. Standard WB testing procedure requires that the buffer, DBS and WB strip are agitated during incubation. Although under current testing procedures it is necessary to submerge each portion of the DBSs and WB strip at least once during each agitation cycle, the DBSs and WB strip need not be completely submerged at all times during incubation. Typically, agitation is generated by rocking the WB tray back and forth about the longitudinal center axis of the tray, also referred to herein as the axis of rotation. Agitation occurs because the rocking motion about the axis of rotation causes the buffer in each trough to move along the longitudinal axis of each trough, which axes are perpendicular to the axis of rotation.

The current WB assay requires the use of two ¼" DBSs per test with enough buffer to submerge the entire length of the WB strip and DBSs at least once during each agitation cycle. For example, if the method of agitation employed during incubation is rocking the WB tray about an axis of rotation which is perpendicular to the longitudinal axes of each trough, the DBSs and length of the strip need to be submerged in the buffer at least once during each cycle.

Also, critical to established WB testing procedures is the maintenance of acceptable concentration ratios of blood sample to buffer. Such procedures presently require two ¼" DBSs and approximately 2 ml of buffer to produce the appropriate concentration when standard WB trays are utilized. Standard WB tray troughs are typically 11.59 cm long and 1.42 cm wide. Assuming these trough dimensions, the height of buffer present in the standard trough should be no less than 0.12 cm in order to completely submerge the DBS(s) and WB strip in buffer once per agitation cycle.

Using current WB testing methods, the DBSs, buffer and WB strip are agitated during incubation for at lease 18 to 22 hours. Accordingly, for large scale testing purposes there exists a need to reduce the agitation/incubation period. Also, because of the limited amount of blood sample available for testing purposes (usually in the form of DBSs), the need also exists for an apparatus and method which requires less blood sample while (1) maintaining an appropriate concentration ratio of dried blood to buffer and (2) keeping the DBS and WB strip submerged in the buffer at least once during each agitation cycle.

### Summary Of The Invention

The present invention provides an improved apparatus and method for reducing the amount of dried blood sample required for performing standard Western Blot testing, particularly for detection of the HIV-1 virus. This invention also provides an improved apparatus and method for reducing the overall incubation cycle time required during the WB test.

Successful performance of the current Cambridge test requires a concentration ratio of blood sample to buffer which equates to approximately two ¼" DBSs and approximately 2 ml by volume of buffer. Commercially available WB trays are provided with typically rectangular troughs. These troughs have a length of approximately 11.59 cm and a width of approximately 1.42 cm. The 2 ml of buffer fills standard troughs to approximately 0.12 cm in height. This height of buffer ensures that the entire WB strip and DBSs are covered at least once during each agitation or rocking cycle.

This invention reduces the amount of blood sample required for WB testing while maintaining or increasing the concentration ratio of dried blood to buffer. These improvements are achieved, at least in part, by altering the shape and dimensions of the troughs found in standard WB trays. Concentrations are maintained or increased by reducing the overall volume of the trough while maintaining the approximate height of liquid buffer so as to assure that the DBSs and WB strip are sufficiently submerged during each agitation cycle. Because less buffer is required in this invention, the blood to buffer concentration may be maintained or increased while less blood sample, (in the form of fewer or smaller DBS(s)) is used. In one embodiment of this invention, the combination of changing the shape and reducing the overall dimensions of the troughs, while using only one ¼" DBS, produces substantially the same test results as when conventional quantities of blood sample and buffer are employed. Additionally, altering the trough geometries, and thereby maintaining or increasing the blood/buffer concentration, may also serve to reduce the incubation time necessary to complete the WB test.

Therefore, the invention disclosed herein provides an apparatus for performing Western Blot testing of blood contained a in dried blood spot. The apparatus comprises a tray having an upper surface. The upper surface has at least one trough formed therein. The trough comprises side walls and a bottom surface which form a dried blood spot receptacle area and an adjacent Western Blot strip receptacle area therein. The trough is also suitable for containing the dried blood spot, a Western Blot strip and liquid buffer.

The invention disclosed also provides a method for performing Western Blot testing of blood contained in a dried blood spot. The method comprises containing a dried blood spot, Western Blot strip and less than approximately 2 ml of liquid buffer. The method also comprises agitating the blood spot, Western Blot strip and buffer in a series of agitation cycles such that each portion of the blood spot and Western Blot strip are substantially submerged in buffer at least once during each agitation cycle. Alternatively, no more than 1 ml of buffer may be contained in the apparatus or utilized in the method.

Accordingly, it is an object of this invention to provide for a Western Blot tray specifically designed to perform testing, such as HIV testing, which minimizes the amount of blood sample required for the test.

It is a further object of this invention to provide for a Western Blot tray that minimizes the volume of buffer required to immerse the entire length of Western Blot strip and DBS(s) at least once during each agitation cycle.

It is yet another object of this invention to provide for a WB tray that minimizes the overall incubation time during the WB test.

These and other objects of the invention will become apparent upon study of the following description of the invention and accompanying drawings.

### Brief Description Of The Drawings

There are shown in these drawings certain preferred embodiments, it being understood that the invention is not limited to the precise embodiments and instrumentalities shown.

Figure 1 is a perspective view of a Western Blot tray in accordance with an embodiment of this invention.

Figure 2 is a plan view of the Western Blot tray of Figure 1.

Figure 3 is an elevation view of the Western Blot tray and agitator of one embodiment of this invention.

Figure 4a is a partial cross-sectional view of an embodiment of the Western Blot tray of this invention, taken along the line 4-4 of Figure 1.

Figure 4b is a partial cross-sectional view of another embodiment of the Western Blot tray of this invention, taken along the line 4-4 of Figure 1.

### Detailed Description

Although the invention disclosed herein may be incorporated into various apparatus and methods, the following detailed description refers to Western Blot testing methods and related apparatus and is not intended to limit the scope of this invention in any way.

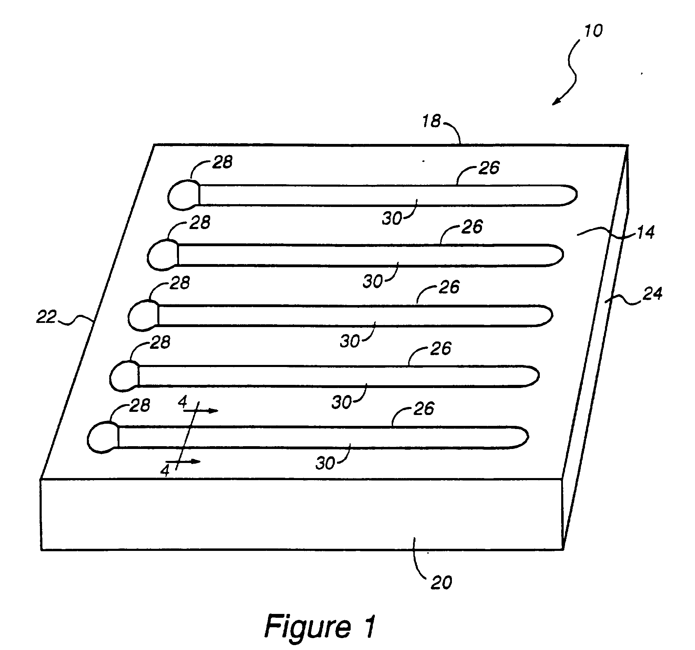

Referring now to the drawings in detail, wherein like reference numerals indicate like elements, there is seen in Figure 1 a WB tray designated by reference numeral 10. Tray 10 comprises an upper surface 14, a lower surface 16, opposite longitudinal side surfaces 18 and 20 and opposite transverse end surfaces 22 and 24, Upper and lower surfaces 14 and 16 are preferably approximately parallel to each other. Side surfaces 18 and 20 preferably are approximately parallel to each other and approximately perpendicular to end surfaces 22 and 24.

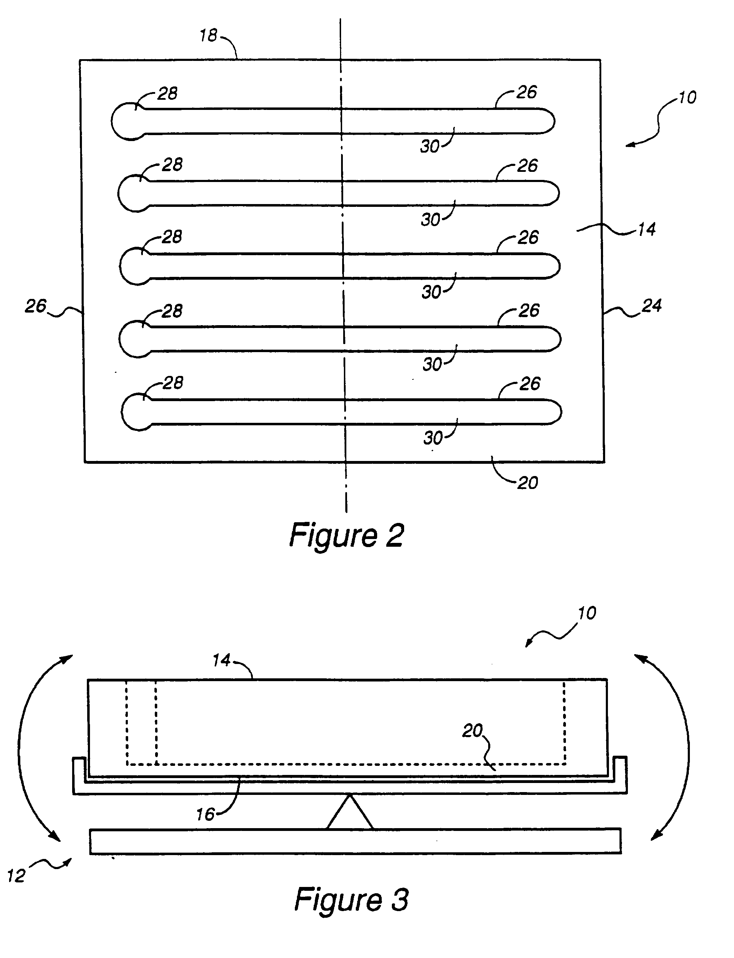

Inasmuch as it is preferable that the buffer, DBS(s) and WB strip contained in each trough are agitated during incubation, there is seen in Figure 3 tray 10 removably mounted to agitator 12. In the embodiment depicted in Figure 3, agitator 12 rocks tray 10 around tray 10's center axis. Tray 10's center axis is shown in Figure 2. The rocking motion about this axis is depicted by the arrows shown in Figure 3.

Turning again to Figure 1, tray 10 may further comprise a cover (not shown) to prevent evaporation, contamination of the buffer and cross-contamination of samples contained in each trough 26. The cover may comprise a component separate from tray 10 itself or it may be hinged or otherwise removably affixed to tray 10.

Referring now to Figures 2 and 3, tray 10 further comprises portions defining at least one trough 26 for containing and reacting at least one DBS, buffer and a WB strip during testing. Preferably, each tray 10 comprises a plurality of troughs 26. However, tray 10 may comprise as few as one trough 26 or as many troughs 26 as may be physically accommodated by tray 10.

In one embodiment of this invention, troughs 26 comprise a DBS receptacle area 28 and a WB strip area 30. DBS area 28 is intended to accommodate the blood sample in the form of a DBS during testing. Strip area 30 is intended to accommodate the WB strip during testing.

The shape of trough 26 is not critical provided the shape reduces the volume of buffer required during testing and the amount of blood sample in the form of DBSs is reduced from the two ¼" DBSs normally used. However, in any configuration of trough 26, a sufficient volume of buffer should preferably be present in trough 26 so that the DBS(s) and all portions of the WB strip are submerged at least once during each agitation cycle.

The shape of the troughs in existing WB trays typically require approximately 2 ml of buffer in order to submerge all areas of the WB strip and DBS during each agitation cycle. In one embodiment of this invention, reduction in the total volume of the trough enables the volume of buffer to be reduced to approximately 1 ml. Reducing trough 26 dimensions enables each portion of the WB strip and DBS(s) to be submerged during each agitation cycle while using approximately 1 ml of buffer. Of course, it is readily apparent that numerous other permutations of trough size, DBS size, and buffer volume are possible in accordance with this invention.

Referring again to Figures 2 and 3, the depth of trough 26 may also vary depending on the geometric limitations needed to maintain sufficient concentration levels and immersion during agitation. However, a preferred depth is approximately 7/8". In the embodiment shown in Figure 1, the volume of trough 26 has been reduced by forming trough 26 into a thermometer shape. The thermometer shaped trough 26 comprises a DBS area 28, which area 28 corresponds to a thermometer bulb, and WB strip area 30, which area corresponds to the body of the thermometer. Bulbed area 28 is shaped to accommodate the DBS(s) while body area 30 is shaped to accommodate the WB strip. Both areas 28 and 30 may be designed to "slip fit" the DBS(s) and WB strip respectively such that trough 26 is slightly larger than the diameter of a DBS at area 28 and slightly larger than the width of the WB strip at area 30. Depending upon the concentration levels desired, the DBS may be sized preferably between about 1/8" and about ¼" in diameter, although it is readily apparent that other sizes and shapes are sufficient. Similarly, the WB strips may be sized preferably between about 1/8" and about ¼"in width, although it is readily apparent that other widths are also acceptable.

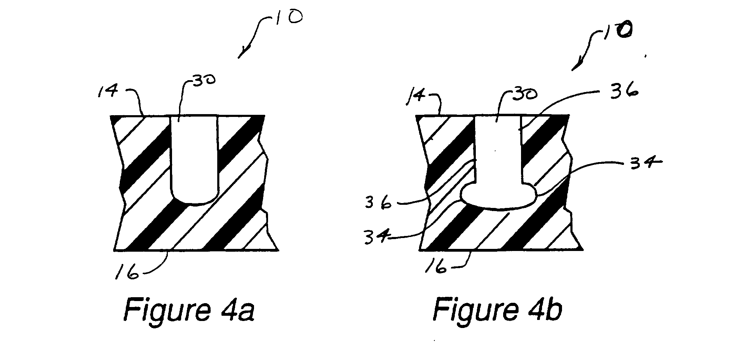

Referring now to Figures 4a and 4b, the shape of the cross section of trough 26 is also not critical so long as (1) the volume of trough 26 allows for complete immersion of the WB strip and DBS(s) at least once during each agitation cycle and (2) a sufficient concentration ratio of blood to buffer is maintained. Cross sections of two preferred embodiments of WB strip area 30 are shown in Figures 4a and 4b respectively. Figure 4a depicts an area 30 of trough 26 with a smooth rounded bottom while Figure 4b depicts area 30 of trough 26 with undercuts 34 and smooth rounded bottom. The undercuts 34 shown in Figure 4b allow for the DBS to move within area 30 during agitation. Area 30 of Figure 4a is preferably about ¼" wide while area 30 of Figure 4b is preferably about 1/8" wide at upper surface 14 and undercut by about 1/8" on either side of the straight sides 36 of area 30 of trough 26 to form undercuts 34. Accordingly, the bottom surface of trough 26 in the undercut embodiment of Figure 4b is approximately 3/8" wide.

Numerous other shapes and configurations of the WB troughs are contemplated which will reduce the volume of the buffer and/or the number and/or size of DBSs required for testing purposes.

The invention disclosed herein will also allow for the utilization of numerous sizes, shapes and quantities of DBSs. In one embodiment of the invention, the amount of blood sample used in the WB test is contained in one ¼" diameter DBS. A ¼" diameter DBS has a surface area of approximately 0.04909 in². In another embodiment, trough 26 geometries are adapted to accommodate one 3/16" diameter DBS or one 1/8" diameter DBS. A 1/8" diameter DBS has a surface area of approximately 0.01227 in² . It should be noted, however, that it is not necessary that the DBS have a circular shape. Any shape of DBS is acceptable provided that the DBS contains a sufficient amount of blood sample (which may correspond to DBS surface area) to assure accurate testing.

Moreover, the WB tray 10 may be modified to accommodate almost any shape of DBS or WB strip. For example, the WB strip may be placed in the WB tray on the WB strip's side or edge. In this embodiment, trough 26 could be approximately 1/8" wide. With the WB strip oriented in this manner, the width of trough 26 would be greatly reduced and consequently, utilize less buffer and a smaller DBS.

Referring again to Figure 3, agitator 12 may comprise any mechanism which provides substantially continuous agitation during the WB incubation period. Agitator 12 may, for example, either rock, tilt or rotate tray 10. Preferably, tray 10 will be rocked, tilted or rotated in one complete cycle once every two to three seconds. One such agitator 12 which uses a rocking motion to create agitation is the Bellco Rocker Platform, Catalog No. 774020000, which measures 50 cm by 50 cm.

Another means for agitating the contents of tray 10 utilizes peristaltic pumps to create a reciprocating flow of the buffer within trough 26. It is expected that any of these agitation means in combination with increased blood to buffer concentrations will reduce incubation times over current WB trays.

It should also be noted that tray 10 is intended to be disposable and preferably made of polystyrene, although other materials may be suitable. Also, tray 10 may be equipped with other optional features. For example, a drain for quick removal or aspiration of buffer from the trough 26 may be provided. It is also possible that a system for automatically filling each trough 26 with buffer may also be provided.

The present invention may be embodied in other specific forms without departing from its spirit or essential attributes. Accordingly, reference should be made to the appended claims rather than to the foregoing specification, for an indication of the scope of this invention.

## Claims

1. An apparatus for performing Western Blot testing of blood contained in a dried blood spot, said apparatus comprising a tray having an upper surface, said upper surface having a trough formed therein, wherein said trough comprises side walls and a bottom surface which form a dried blood spot receptacle area and an adjacent Western Blot strip receptacle area therein, and wherein said trough is suitable for containing said dried blood spot, a Western Blot strip and liquid buffer.

2. The tray of claim 1 wherein said Western blot strip receptacle area comprises straight side walls and a bottom surface.

3. The tray of claim 2 wherein said Western Blot strip receptacle area further comprises side walls having undercut regions adjacent to said bottom surface.

4. The tray of claim 1 further comprising a plurality of said troughs.

5. The tray of claim 2 further comprising a plurality of said troughs.

6. The tray of claim 3 further comprising a plurality of said troughs.

7. The tray of claim 1 further comprising a means for agitating said dried blood spot, Western Blot strip and buffer contained in said trough.

8. An apparatus for performing Western Blot testing of blood contained in a dried blood spot, said apparatus comprising:
means for containing said dried blood spot, a Western Blot strip and less than approximately 2 ml of liquid buffer; and
means for agitating said blood spot, Western Blot strip and buffer in a series of agitation cycles such that each portion of said blood spot and Western Blot strip are substantially submerged in buffer at least once during each agitation cycle.

9. The apparatus of claim 8 wherein said apparatus comprises a plurality of means for containing a blood spot, a Western Blot strip and liquid buffer.

10. A method for performing Western Blot testing of blood contained in a dried blood spot, said method comprising the steps of:
containing a dried blood spot, Western Blot strip and less than approximately 2 ml of liquid buffer; and
agitating said blood spot, Western Blot strip and buffer in a series of agitation cycles such that each portion of said blood spot and Western Blot strip are substantially submerged in buffer at least once during each agitation cycie.
